# EUROPEAN PATENT SPECIFICATION

(11) **EP 4 023 745 B1**
(45) Date of publication and mention of the grant of the patent: **08.10.2025**
(21) Application number: 20857703.1
(22) Date of filing: 26.08.2020
(51) Int. Cl.: C12M 1/26, C12Q 1/24, G01N 1/04, G01N 33/15, G01N 33/48, G01N 33/50, A61B 10/00, A61B 10/02, C12Q 1/02, C40B 30/06, G01N 1/28, G01N 33/569, G01N 33/68, G01N 1/02

(54) **ADHESIVE TAPE FOR COLLECTING SKIN MICROORGANISMS OR SKIN METABOLITES**
KLEBEBAND ZUM SAMMELN VON HAUTMIKROORGANISMEN ODER HAUTMETABOLITEN
RUBAN ADHÉSIF POUR PRÉLÈVEMENT DE MICROORGANISMES INDIGÈNES DE LA PEAUOU DE MÉTABOLITE DE LA PEAU

(30) Priority: 26.08.2019 JP 2019154041
(43) Date of publication of application: 06.07.2022
(73) Proprietor: MySkin Corporation, Tokyo 1130033 (JP); The University of Tokyo, Bunkyo-ku Tokyo 113-8654 (JP)
(72) Inventor: INOUE Fukashi, Tokyo 113-0033 (JP); TACHIBANA Kouta, Tokyo 113-0033 (JP); SUTANI Takashi, Tokyo 113-8654 (JP)
(74) Representative: Appleyard Lees IP LLP
(86) International application number: PCT/JP2020/032217
(87) International publication number: WO 2021/039854

(56) References cited:
- WO-A1-2018/043636
- WO-A1-2018/043636
- WO-A1-2018/161062
- AU-A1- 2014 281 553
- CN-A- 107 773 275
- JP-A- 2001 161 697
- JP-A- 2001 161 697
- JP-A- 2015 193 597
- JP-A- S63 106 558
- JP-B1- 6 046 849
- JP-B2- S6 046 849
- JP-U- 3 142 919
- ANONYMOUS: "POLA Chemical thoroughly investigates men's skin The shine of the forehead increases significantly from 4 hours after washing the face, etc.", NEWS RELEASE P O HOLDINGS, 18 March 2009 (2009-03-18), pages 1 - 3, XP055916086

## Description

### TECHNICAL FIELD

The present disclosure relates to an adhesive tape for collecting skin indigenous microorganisms, a method of evaluating a physical condition of a subject, a method of presenting information to a subject, and an adhesive tape for collecting skin metabolites. Also disclosed is a method of screening for a substance capable of improving or preventing a physical condition.

### BACKGROUND ART

Conventionally, human skin conditions are evaluated using indices such as resilience, texture, spots, wrinkles, the level of moisture, the level of oiliness, and the amount of moisture transpiration (barrier function). Methods of evaluating these indices include questionnaire studies, image analysis of the skin surface disclosed in Patent Document 1 and the like (for resilience, texture, spots, wrinkles, and the like), and instrumental measurements (for the level of moisture, the level of oiliness, the amount of moisture transpiration, and the like).

However, questionnaire studies may suffer from significant bias due to questionee's subjectivity, and tend to yield less objective evaluations. Unlike questionnaire studies, image analysis can provide objective indices. Nonetheless, accurate evaluation of skin conditions may still be difficult due to varied skin qualities depending on whether the face has been cleansed, application of foundation, skin cream, and the like.

Further, evidence has been accumulating which suggests that factors affecting skin conditions may not necessarily be limited to the aforementioned indices, and there exists a skin condition difficult to be evaluated by the conventional technologies which are dependent on the aforementioned indices. There are increasing demands for a technology allowing such a skin condition to be evaluated. In addition to such a skin condition, there also exist demands for a technology allowing a general physical condition to be evaluated.

In view of such circumstances, the present applicants developed, as a technology to evaluate a skin condition that can yield objective evaluations and is less affected by varied skin qualities due to face cleansing and the like, a method of evaluating a physical condition of a subject, the method including the step of: determining a value of an abundance proportion of a skin indigenous microorganism on a skin surface of the subject among a skin microbial flora collected from the skin surface or a parameter based on the abundance proportion, a correlation criterion of the abundance proportion or parameter with the physical condition being pre-established, and then comparing the value with the correlation criterion, wherein the physical condition is a skin condition being at least one selected from the group consisting of skin age; oily skin; and spots, dullness, sagging, wrinkles, resilience conditions, and pore conditions of the skin, or a condition relating to hair, teeth, urine, bone, internal organs, blood, urine, or nerve (see Patent Document 2).

Patent Document 1: Japanese Unexamined Patent Application, Publication No. 2013-188326
Patent Document 2: Japanese Patent No. 6046849

Non-Patent Document 1: Zouboulis, C.C., Stratakis, C.A., Chrousos, G.P. et al. Metabolism and skin diseases. Rev Endocr Metab Disord 17, 241-246 (2016).
JP2001161697 refers to an adhesive tape for collecting skin epidermic cells. The adhesive tape comprises a base material and an adhesive layer on the base material and the tape has an adhesive power of 10 - 50 N/25mm.
CN107773275 refers to a skin detection sampling package comprising an adhesive tape to remove keratinocytes from the skin surface.

### DISCLOSURE OF THE INVENTION

### Problems to be Solved by the Invention

The present invention is described in the appended claims. In the technology described in Patent Document 2, it is required to collect skin indigenous microorganisms from a skin surface of a subject. Methods for collecting skin indigenous microorganisms from a skin surface of a subject include a collection method in a wet mode with use of a cotton bud (swab). This method allows skin indigenous microorganisms on a skin surface to be collected in a satisfactory manner, but suffers from problems of unstable manipulation for collection and requirement of refrigerated or frozen storage for satisfactory preservation of collected samples. Contemplated as a countermeasure is a method of collecting skin indigenous microorganisms from a skin surface in a dry mode with use of a tape. With this method, however, it is difficult to collect skin indigenous microorganisms on a skin surface in a satisfactory manner. Since the amounts of skin indigenous microorganisms on a skin surface are very small, it is desired for evaluation and the like with high accuracy to collect skin indigenous microorganisms on a skin surface in a satisfactory manner.

If skin metabolites are successfully collected from a skin surface, the skin metabolites may allow the physical condition to be evaluated (see Non-Patent Document 1). Thus, it is also desirable to collect skin metabolites on a skin surface in a satisfactory manner.

The present invention is made in view of the aforementioned traditional circumstances. An object of the present invention is to provide: an adhesive tape for collecting skin indigenous microorganisms or skin metabolites, the adhesive tape being capable of providing improved collection yields of skin indigenous microorganisms on a skin surface; and a method of analysing a sample. Disclosed herein, although not forming part of the present invention is a method of screening for a substance capable of improving or preventing a physical condition. Each method uses the adhesive tape of the present invention.

### Means for Solving the Problems

The present inventors found that an adhesive tape for collecting skin indigenous microorganisms, the adhesive tape including an adhesive layer containing a rubber adhesive and having specific adhesive strength and peel strength, provides improved collection yields of skin indigenous microorganisms on a skin surface. Then the present invention has been completed. In addition, the present inventors found that an adhesive tape for collecting skin metabolites, the adhesive tape including an adhesive layer containing a rubber adhesive and having specific adhesive strength and peel strength, allows skin metabolites on a skin surface to be collected in a satisfactory manner.

### Effects of the Invention

The present invention can provide an adhesive tape for collecting skin indigenous microorganisms, the adhesive tape being capable of providing improved collection yields of skin indigenous microorganisms on a skin surface. Further, the present invention can provide an adhesive tape for collecting skin metabolites, the adhesive tape allowing skin metabolites on a skin surface to be collected in a satisfactory manner. Furthermore, with use of the adhesive tape for collecting skin indigenous microorganisms or the adhesive tape for collecting skin metabolites, the present invention can provide a method of evaluating a physical condition of a subject, and a method of presenting information to a subject. Disclosed herein is a method of screening for a substance capable of improving or preventing a physical condition. Each method has high accuracy.

### BRIEF DESCRIPTION OF THE DRAWINGS

Fig. 1 shows a perspective view illustrating an example of the adhesive tape for collecting skin indigenous microorganisms according to the present invention.
Fig. 2 shows an operation flow chart for an example of the method of measuring a bacterial flora with use of the adhesive tape for collecting skin indigenous microorganisms according to the present invention.
Fig. 3 shows a schematic diagram illustrating a method of measuring adhesive strength.
Fig. 4 shows a schematic diagram illustrating a method of measuring peel strength.
Fig. 5 shows results of measurement of total DNA content for a subject A.
Fig. 6 shows results of measurement of total DNA content for a subject B.
Fig. 7 shows results of measurement of total DNA content for a subject C.
Fig. 8 shows results of measurement of total DNA content for a subject D.
Fig. 9 shows results of measurement of total DNA content for a subject E.

### PREFERRED MODE FOR CARRYING OUT THE INVENTION

Below, the present invention will be specifically described.

### <Adhesive tape for collecting skin indigenous microorganisms>

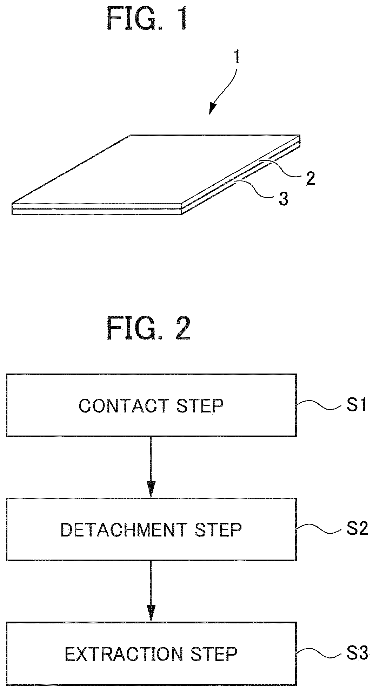

The adhesive tape for collecting skin indigenous microorganisms according to the present invention is an adhesive tape for collecting skin indigenous microorganisms for use in collecting a skin indigenous microorganism on a skin surface, and includes: a base material; and an adhesive layer provided on at least a portion of a surface of the base material. The adhesive layer contains an adhesive, and the adhesive contains a rubber adhesive. The adhesive tape for collecting skin indigenous microorganisms has an adhesive strength of 10.0 N or more when a 12 mm-wide test piece thereof is pressure-bonded to a Bakelite plate by one reciprocation of a 2 kg roller and then measured for adhesive strength at a pulling speed of 300 mm/min and a pulling angle of 90° according to JIS Z 0237: 2009, and the adhesive tape has a peel strength of 0.25 N or more when a 12 mm-wide test piece thereof is measured using a silicone-treated fine paper sheet as a release sheet and measured for peel strength at a pulling speed of 300 mm/min and a pulling angle of 180° according to JIS Z 0237: 2009. The presented adhesive tape for collecting skin indigenous microorganisms according to the present invention will be described in detail with reference to Fig. 1. Fig. 1 shows a perspective view illustrating an example of the adhesive tape for collecting skin indigenous microorganisms according to the present invention.

As illustrated in Fig. 1, the adhesive tape 1 for collecting skin indigenous microorganisms according to the present invention includes: a base material 2; and an adhesive layer 3 provided on a surface of the base material 2. Although Fig. 1 shows the case that the adhesive layer 3 is provided on the entire of one surface of the base material 2, it is sufficient for the adhesive layer 3 to be provided on at least a portion of the surface of the base material 2. For example, the base material 2 may be exposed with the adhesive layer 3 being absent on a portion of the surface of the base material 2.

The adhesive layer 3 contains an adhesive, and the adhesive contains a rubber adhesive. The rubber adhesive contained in the adhesive layer 3 may be one or two or more selected from the group consisting of natural rubbers and synthetic rubbers, but it is preferred for the adhesive layer 3 to contain a natural rubber.

The adhesive tape for collecting skin indigenous microorganisms according to the present invention has an adhesive strength of 10.0 N or more when a 12 mm-wide test piece thereof is pressure-bonded to a Bakelite plate by one reciprocation of a 2 kg roller and then measured for adhesive strength at a pulling speed of 300 mm/min and a pulling angle of 90° according to JIS Z 0237: 2009 (hereinafter, which may be referred to as "adhesive strength", simply). The adhesive strength is preferably 11.0 N or more, more preferably 12.0 N or more, and even more preferably 13.0 N or more. There is no particular limitation for the upper limit of the adhesive strength, but the adhesive strength is, for example, 20.0 N or less, preferably 19.0 N or less, more preferably 18.0 N or less, and even more preferably 17.0 N or less. The adhesive tape for collecting skin indigenous microorganisms according to the present invention has a peel strength of 0.25 N or more when a 12 mm-wide test piece thereof is measured using a silicone-treated fine paper sheet as a release sheet and measured for peel strength at a pulling speed of 300 mm/min and a pulling angle of 180° according to JIS Z 0237: 2009 (hereinafter, which may be referred to as "peel strength", simply). The peel strength is preferably 0.26 N or more, more preferably 0.27 N or more, and even more preferably 0.28 N or more. There is no particular limitation for the upper limit of the peel strength, but the peel strength is, for example, 0.50 N or less, preferably 0.45 N or less, more preferably 0.40 N or less, and even more preferably 0.35 N or less.

Use of an adhesive tape for collecting skin indigenous microorganisms, the adhesive tape including, as described above, the adhesive layer 3 containing a rubber adhesive and having an adhesive strength of 10.0 N or more and a peel strength of 0.25 N or more, can provide improved collection yields of skin indigenous microorganisms on a skin surface compared with those provided by conventional tapes, as demonstrated later with Example and Comparative Examples. Accordingly, collecting a skin indigenous microorganism on a skin surface with use of such an adhesive tape for collecting skin indigenous microorganisms, the adhesive tape including an adhesive layer containing a rubber adhesive and having an adhesive strength of 10.0 N or more and a peel strength of 0.25 N or more, is as favorable as collecting a skin indigenous microorganism on a skin surface, for example, in a wet mode with use of a cotton bud (swab) that allows skin indigenous microorganisms on a skin surface to be collected in a satisfactory manner. Moreover, such collection with a tape makes manipulation for collection easy.

There is no particular limitation for the thickness of the adhesive tape for collecting skin indigenous microorganisms according to the present invention. The thickness is, for example, 0.01 mm or larger, and may be 0.10 mm or larger or 0.20 mm or larger. Likewise, there is no particular limitation for the upper limit of the thickness. The thickness is, for example, 0.50 mm or smaller, and may be 0.40 mm or smaller or 0.30 mm or smaller.

There is no particular limitation for the location from which a skin microbial flora is collected with the adhesive tape for collecting skin indigenous microorganisms according to the present invention, and examples of the location include the face, scalp, armpit, and navel. Among these, the face, in particular the forehead is preferred. It is preferred to use the adhesive tape for collecting skin indigenous microorganisms according to the present invention for collecting a skin indigenous microorganism on a skin surface having a sebum level of 75 or higher. The adhesive tape for collecting skin indigenous microorganisms according to the present invention can provide highly improved collection yields of skin indigenous microorganisms on a skin surface having a sebum level of 75 or higher. Needless to say, the adhesive tape for collecting skin indigenous microorganisms may be used for collecting a skin indigenous microorganism on a skin surface having a sebum level of lower than 75. The sebum level of a skin surface of a subject varies depending on the environment and the body condition or the like. Thus, collection can be performed for a wide variety of subjects even when a skin indigenous microorganism on a skin surface having a sebum level of 75 or higher is to be collected.

There is no particular limitation for the skin indigenous microorganism to be collected with the adhesive tape for collecting skin indigenous microorganisms according to the present invention. The skin indigenous microorganism may be a bacterium or a fungus, but a bacterium is particularly preferred. Specific examples of skin indigenous microorganisms include bacteria belonging to phylum Actinobacteria, bacteria belonging to phylum Proteobacteria, and bacteria belonging to phylum Firmicutes, and bacterium Propionibacterium acnes (P. acnes) (also called Cutibacterium acnes, in recent years).

In storing the adhesive tape for collecting skin indigenous microorganisms according to the present invention after collecting skin indigenous microorganisms on a skin surface with the adhesive tape for collecting skin indigenous microorganisms (for example, in the case that skin indigenous microorganisms on a skin surface are collected with the adhesive tape for collecting skin indigenous microorganisms and after a while the collected skin microbial flora is analyzed), there is no particular limitation for the temperature to store the adhesive tape for collecting skin indigenous microorganisms, and the adhesive tape for collecting skin indigenous microorganisms may be preserved at normal temperature, or preserved under refrigeration or freezing.

### <Applications of adhesive tape for collecting skin indigenous microorganisms>

The adhesive tape for collecting skin indigenous microorganisms according to the present invention can be used for a method of evaluating a physical condition of a subject. The method of evaluating a physical condition of a subject includes the step of: determining a value of an abundance proportion of a skin indigenous microorganism on a skin surface of the subject among a skin microbial flora collected from the skin surface or a parameter based on the abundance proportion, a correlation criterion of the abundance proportion or parameter with the physical condition being pre-established, and then comparing the value with the correlation criterion, and the skin microbial flora collected from the skin surface of the subject is a skin microbial flora obtained by collecting a skin indigenous microorganism on the skin surface by using the aforementioned adhesive tape for collecting skin indigenous microorganisms according to the present invention.

This enables various physical conditions to be accurately evaluated. Further, certain physical conditions which have been traditionally recognized in a collective manner may be subdivided by virtue of the abundance proportion or parameter of a skin indigenous microorganism, thereby improving a physical condition of a subject in a more reliable fashion. In addition, use of the aforementioned adhesive tape for collecting skin indigenous microorganisms provides improved collection yields of skin indigenous microorganisms, and thus enables evaluation of physical conditions with high accuracy.

Further, the adhesive tape for collecting skin indigenous microorganisms according to the present invention can be used for a method of presenting information to a subject. The method of presenting information to a subject is a method including the steps of: evaluating a physical condition of the subject by the aforementioned method of evaluating a physical condition of a subject, and presenting, when the physical condition is evaluated to be outside a target condition, information registered in a data base as an ingredient capable of directing the value of the abundance proportion or the parameter toward a numerical range of the abundance proportion or parameter obtained based on the correlation criterion when the physical condition is within the target condition. This enables information for improving a physical condition and the like to be personalized and presented to a subject. It is noted that the term "information" as used herein encompasses the identity, directions, and dosage of an ingredient or a formulation containing the ingredient, the way and extent of expected improvement in a physical condition, and an adverse effect of the ingredient or formulation.

In the method of presenting information to a subject, the followings are preferably further included: a step of re-evaluating the physical condition of the subject after consumption of the ingredient by the aforementioned method of evaluating a physical condition of a subject; and updating the information based on the extent of improvement in the physical condition after the re-evaluation. Thereby, the information in the data base can be improved, leading to more reliable improvement of a physical condition and others.

There is no particular limitation for the timing of re-evaluation, but, for example, the re-evaluation may be performed when a period of time likely enough to improve a physical condition in response to the use of an ingredient has passed, which may be included in the information in the data base.

Furthermore, disclosed herein, although not forming part of the present invention, is the use of the adhesive tape for collecting skin indigenous microorganisms in a method of screening for a substance capable of improving or preventing a physical condition. The method of screening for a substance capable of improving or preventing a physical condition includes the step of: selecting a candidate substance based on how the candidate substance changes a value of an abundance proportion of a skin indigenous microorganism on a skin surface or a parameter based on the abundance proportion, a correlation criterion of the abundance proportion or parameter with the physical condition being pre-established, and the skin microbial flora collected from the skin surface of the subject is a skin microbial flora obtained by collecting a skin indigenous microorganism on the skin surface by using the aforementioned adhesive tape for collecting skin indigenous microorganisms. This can likely increase a probability of screening for a substance capable of improving or preventing a physical condition.

It is noted that the term "screening" means that the probability of the presence of a substance capable of improving or preventing a physical condition is higher in the group of candidate substances after the screening method than in those before the screening method, but a candidate substance after the screening method may not necessarily be actually capable of improving or preventing a physical condition. Therefore, it is preferred to further perform a step of examining whether a screened candidate substance can actually improve or prevent a physical condition.

The aforementioned methods will be more specifically described.

The skin indigenous microorganism may be a bacterium or a fungus, but a bacterium is particularly preferred. The abundance proportion thereof may be in terms of any of phylum, class, order, family, genus, or species. For example, it may be an abundance proportion of at least one bacterium selected from the group consisting of bacteria belonging to phylum Actinobacteria, bacteria belonging to phylum Proteobacteria, and bacteria belonging to phylum Firmicutes. Among these, the abundance proportion of bacterium P. acnes may be mentioned.

There is no particular limitation for the parameter as long as it is based on the abundance proportion of a skin indigenous microorganism. The parameter may be the difference, sum, product, ratio, exponent, or logarithm of the abundance proportions of a plurality of skin indigenous microorganisms, or a combination thereof. In particular, the parameter may be the ratio, difference, or a combination thereof. For example, the difference in the relative amounts of a bacterial species belonging to phylum Proteobacteria and a bacterial species belonging to phylum Firmicutes may be mentioned. Alternatively, the parameter may be a diversity index of skin indigenous microorganisms. Such parameters may be each a parameter correlating with a coordinate axis when data for specimens in a population are plotted on a coordinate space by using a technique of dimensionality reduction (for example, principal coordinate analysis, principal component analysis).

There is no particular limitation for the physical condition as long as it shows correlation with the abundance proportion of a skin indigenous microorganism on a skin surface or a parameter based on the abundance proportion, but it can be a condition related to any part such as skin, hair, teeth, urine, bone, internal organs, blood, and nerve. Skin conditions include skin age; oily skin; pimpled skin; freckles, texture, yellowishness, dryness, shininess, spots, dullness, sagging, wrinkles, resilience conditions, and pore conditions of the skin; yellowish skin; and the like. Hair conditions include thin hair, hair loss, and the like. Teeth conditions include periodontal disease, alveolar pyorrhea, and the like. Conditions of bone, internal organs, blood, urine, and nerve include liver functions, high uric acid, knee pain, osteoporosis, autonomic imbalance, diabetes, and the like. In addition, the physical condition encompasses menopause which may affect female hormone and aesthetic properties.

The term "correlation criterion" refers to a criterion which can qualitatively or quantitatively determine the goodness or seriousness of a physical condition to be evaluated based on the value of the aforementioned abundance proportion or parameter determined for a skin microbial flora.

Results from analysis of bacterial floras by the present inventors show that, as demonstrated in Patent Document 2, the relative amount of a bacterium belonging to phylum Actinobacteria and the abundance ratio of a bacterium belonging to phylum Proteobacteria and a bacterium belonging to phylum Firmicutes, which are specific indigenous bacteria present on a facial skin surface, may be determined to evaluate various facial skin conditions such as pimples and expanded pores and susceptibility to various physical symptoms not localized to the face such as periodontal disease and thin hair/hair loss in the head.

Further, the relative amount of a bacterial species may correlate with susceptibility to other physical symptoms. For example, the followings and others show a correlation with the relative amount of a bacterial species indicated in parentheses: high uric acid values (genus Rothia, genus Brevibacterium, family Erythrobacteraceae, and others), osteoporosis (genus Pseudomonas, genus Methylobacterium), decreased liver function (genus Campylobacter and others), yellowish skin (genus Bacillus, bacterium Rothia dentocariosa, and others), diabetes (a bacterium belonging to order Actinomycetales).

The aged skin index, which is computed from the presence or absence of sagging, wrinkles, non-resilience, and the like of the facial skin, shows a correlation with the abundance of a series of bacteria which may be referred to as "aged skin bacteria," including bacterial species belonging to class Gammaproteobacteria, bacterium Propionibacterium granulosum, and others.

Many of physical conditions may correlate with not only a skin microbial flora but also an extra phenomenon (for example, actual age). For this reason, an effect from at least one phenomenon known to correlate with a physical condition is preferably removed from the correlation criterion. This can reduce an effect from the presence or absence and the extent of the aforementioned extra phenomenon about a subject, enabling more accurate evaluation of a physical condition based on the value of the abundance proportion or parameter of a skin microbial flora. Further, according to the method of presenting information to a subject, more suitable information can be presented considering the presence or absence and the extent of the above extra phenomenon about a subject. For this reason, the presence or absence and the extent of the above extra phenomenon about the subject is preferably input in addition to the abundance proportion or parameter. Further, in the method of screening for a substance capable of improving or preventing a physical condition, a substance which may improve or prevent a physical condition tends to be obtained more easily regardless of the above extra phenomenon about a user.

Moreover, the correlation criterion may be created for a sub-population into which a population is categorized according to at least one selected from the group consisting of age, sex, residential area, nationality, and race. By this, a physical condition can be accurately evaluated even considering the age, sex, residential area, nationality, race, and the like of a subject. Further, according to the method of presenting information to a subject, more suitable information can be presented considering the age, sex, residential area, nationality, and race of the subject. For this reason, at least one selected from the group consisting of the age, sex, residential area, nationality, and race of a subject is preferably input in addition to the abundance proportion or parameter. Moreover, in the method of screening for a substance capable of improving or preventing a physical condition, a substance which may improve or prevent a physical condition and be suitable for the age, sex, residential area, nationality, and race of a user tends to be obtained more easily.

There is no particular limitation for the location from which a skin microbial flora is collected, but it may be the face, scalp, armpit, navel, and the like. Among these, the face, in particular the forehead can be mentioned. The location from which a skin microbial flora is collected is preferably a skin surface having a sebum level of 75 or higher. This is because the aforementioned adhesive tape for collecting skin indigenous microorganisms can provide highly improved collection yields of skin indigenous microorganisms on a skin surface having a sebum level of 75 or higher. Needless to say, the location from which a skin microbial flora is collected may have a sebum level of lower than 75. The sebum level of a skin surface of a subject varies depending on the environment and the body condition or the like. Thus, collection can be performed for a wide variety of subjects even when the location from which a skin microbial flora is collected is set to a skin surface having a sebum level of 75 or higher. It is noted that a location from which a sample is collected for a subject preferably coincides with a location from which samples were collected for a population where a correlation criterion was created.

Determination of values for a skin microbial flora collected from a skin surface of a subject is carried out by analyzing a skin microbial flora collected from a skin surface of a subject by using the aforementioned adhesive tape for collecting skin indigenous microorganisms according to the present invention.

A bacterial flora on a skin surface is preferably analyzed by analyzing DNA present on the skin surface. The method of investigating a bacterial flora based on DNA can yield results more accurately reflecting the bacterial flora as compared with the method of estimating a bacterial flora by culturing bacteria collected from a skin surface on an agar medium. This is because the percentage of microorganisms cultivable on an agar medium is thought to be only about 1%, and most microbial species are difficult to be cultured. In contrast, the method involving analysis of DNA enables a bacterial flora collected from a skin surface to be analyzed accurately and truly.

Such analysis of a bacterial flora is preferably performed by a method including:
a detachment step of detaching the adhesive tape 1 for collecting skin indigenous microorganisms the adhesive layer 3 of which has been contacted with a skin surface to allow bacteria present on the skin surface to adhere to the adhesive layer,
an extraction step of extracting DNA contained in the bacteria by allowing the adhesive layer 3 to which the bacteria has been adhered to make contact with a liquid, and a genome analysis step of performing genome analysis of the extracted DNA.

In the aforementioned method of analyzing a bacterial flora, the adhesive layer 3 of the adhesive tape 1 for collecting skin indigenous microorganisms is contacted with a skin surface, and then detached from the skin surface to collect bacteria present on the skin surface while the bacteria remain adhering to the adhesive layer 3 (the detachment step). Thereby, amongst the entire skin surface bacteria, those present on a skin surface with which the adhesive layer 3 makes contact can be allowed to adhere to and collected at a surface of the adhesive layer 3 of the adhesive tape 1 for collecting skin indigenous microorganisms while maintaining the original distribution. Advantageously, the aforementioned method can eliminate a risk of variations in the data of a bacterial flora due to varied swabbing force and varied swabbing area in the method of swabbing a skin surface with a swab. Further, the bacterial flora of a skin surface can be truly copied. Subsequently, the adhesive layer 3 having the bacteria adhered is allowed to make contact with a liquid to extract DNA included in bacteria (the extraction step). Therefore, a DNA solution, from which a bacterial flora can be determined, can be prepared very rapidly from the adhesive member having the bacteria adhered as compared with a method of sorting substances which have adhered to the surface of an adhesive layer using a magnifying glass and the like to prepare a sample. This can minimize a change in the bacterial flora during the course of sample preparation. Then, the extracted DNA can be analyzed with the next-generation sequencer (NGS) and the like to simply and uniformly determine the bacterial flora present on the skin surface. There is also no particular limitation for the means for extracting DNA and the means for analyzing DNA.

### <Adhesive tape for collecting skin metabolites>

The adhesive tape for collecting skin metabolites according to the present invention is an adhesive tape for collecting skin metabolites for use in collecting a skin metabolite on a skin surface, and includes: a base material; and an adhesive layer provided on at least a portion of a surface of the base material. The adhesive layer contains an adhesive, and the adhesive contains a rubber adhesive. The adhesive tape for collecting skin metabolites has an adhesive strength of 10.0 N or more when a 12 mm-wide test piece thereof is pressure-bonded to a Bakelite plate by one reciprocation of a 2 kg roller and then measured for adhesive strength at a pulling speed of 300 mm/min and a pulling angle of 90° according to JIS Z 0237: 2009. The adhesive tape for collecting skin metabolites has a peel strength of 0.25 N or more when a 12 mm-wide test piece thereof is measured using a silicone-treated fine paper sheet as a release sheet and measured for peel strength at a pulling speed of 300 mm/min and a pulling angle of 180° according to JIS Z 0237: 2009. The presented adhesive tape for collecting skin metabolites according to the present invention has the same configuration as the aforementioned adhesive tape 1 for collecting skin indigenous microorganisms has.

The adhesive tape for collecting skin metabolites according to the present invention includes: a base material; and an adhesive layer provided on a surface of the base material. It is sufficient for the adhesive layer to be provided on at least a portion of the surface of the base material. For example, the adhesive layer may be provided on the entire of one surface of the base material, and the base material may be exposed with the adhesive layer being absent on a portion of the surface of the base material.

The adhesive layer contains an adhesive, and the adhesive contains a rubber adhesive. The rubber adhesive contained in the adhesive layer may be one or two or more selected from natural rubbers and synthetic rubbers, but it is preferred for the adhesive layer to contain a natural rubber.

The adhesive tape for collecting skin metabolites according to the present invention has an adhesive strength of 10.0 N or more when a 12 mm-wide test piece thereof is pressure-bonded to a Bakelite plate by one reciprocation of a 2 kg roller and then measured for adhesive strength at a pulling speed of 300 mm/min and a pulling angle of 90° according to JIS Z 0237: 2009 (hereinafter, may be referred to as " adhesive strength", simply). The adhesive strength is preferably 11.0 N or more, more preferably 12.0 N or more, and even more preferably 13.0 N or more. There is no particular limitation for the upper limit of the adhesive strength, but the adhesive strength is, for example, 20.0 N or less, preferably 19.0 N or less, more preferably 18.0 N or less, and even more preferably 17.0 N or less. The adhesive tape for collecting skin metabolites according to the present invention has a peel strength of 0.25 N or more when a 12 mm-wide test piece thereof is measured using a silicone-treated fine paper sheet as a release sheet and measured for peel strength at a pulling speed of 300 mm/min and a pulling angle of 180° according to JIS Z 0237: 2009 (hereinafter, may be referred to as "peel strength", simply). The peel strength is preferably 0.25 N or more, more preferably 0.27 N or more, and even more preferably 0.28 N or more. There is no particular limitation for the upper limit of the peel strength, but the peel strength is, for example, 0.50 N or less, preferably 0.45 N or less, more preferably 0.40 N or less, and even more preferably 0.35 N or less.

Use of an adhesive tape for collecting skin metabolites, the adhesive tape including, as described above, an adhesive layer containing a rubber adhesive and having an adhesive strength of 10.0 N or more and a peel strength of 0.25 N or more, allows metabolites on a skin surface, specifically, metabolites on the skin or metabolites derived from the interior of the skin on a skin surface to be collected in a satisfactory manner. In addition, use of the adhesive tape for collecting skin metabolites provides significantly higher collection yields of metabolites on a skin surface than use of any conventional tape provides. Accordingly, collecting a skin metabolite on a skin surface with use of such an adhesive tape for collecting skin metabolites, the adhesive tape including an adhesive layer containing a rubber adhesive and having an adhesive strength of 10.0 N or more and a peel strength of 0.25 N or more, is as favorable as collecting a skin metabolite on a skin surface, for example, in a wet mode with use of a cotton bud (swab) that allows skin metabolites on a skin surface to be collected in a satisfactory manner. Moreover, such collection with a tape makes manipulation for collection easy.

There is no particular limitation for the thickness of the adhesive tape for collecting metabolites according to the present invention. The thickness is, for example, 0.01 mm or larger, and may be 0.10 mm or larger or 0.20 mm or larger. Likewise, there is no particular limitation for the upper limit of the thickness. The thickness may be, for example, 0.50 mm or smaller, and may be 0.40 mm or smaller or 0.30 mm or smaller.

There is no particular limitation for the location from which a skin metabolite is collected with the adhesive tape for collecting skin metabolites according to the present invention, and examples of the location include the face, scalp, armpit, and navel. Among these, the face, in particular the forehead is preferred. It is preferred to use the adhesive tape for collecting skin metabolites according to the present invention for collecting a skin metabolite on a skin surface having a sebum level of 75 or higher. The adhesive tape for collecting skin metabolites according to the present invention can provide highly improved collection yields of skin metabolites on a skin surface having a sebum level of 75 or higher. Needless to say, the adhesive tape for collecting skin metabolites according to the present invention may be used for collecting a skin metabolite on a skin surface having a sebum level of lower than 75. The sebum level of a skin surface of a subject varies depending on the environment and the body condition or the like. Thus, collection can be performed for a wide variety of subjects even when a skin metabolite on a skin surface having a sebum level of 75 or higher is to be collected.

There is no particular limitation for the skin metabolite to be collected with the adhesive tape for collecting skin metabolites according to the present invention. The skin metabolite is, for example, a soluble protein. In an embodiment of the present invention, the total amount of soluble proteins can be measured, for example by biochemical quantitative analysis (such as a BCA method). Identification and quantification of individual soluble proteins can be carried out, for example, by a biochemical technique with an antibody (such as Western blotting and ELISA) or by a mass spectrometer.

In storing the adhesive tape for collecting skin metabolites according to the present invention after collecting skin metabolites on a skin surface with the adhesive tape for collecting skin metabolites (for example, in the case that skin metabolites on a skin surface are collected with the adhesive tape for collecting skin metabolites and after a while the collected skin metabolites are analyzed), there is no particular limitation for the temperature to store the adhesive tape for collecting skin metabolites, and the adhesive tape for collecting skin metabolites may be preserved at normal temperature, or preserved under refrigeration or freezing.

### <Applications of adhesive tape for collecting skin metabolites>

The adhesive tape for collecting skin metabolites according to the present invention can be used for a method of evaluating a physical condition of a subject. The method of evaluating a physical condition of a subject includes the step of: determining a value of an abundance proportion of a skin metabolite on a skin surface of the subject among skin metabolites collected from the skin surface or a parameter based on the abundance proportion, a correlation criterion of the abundance proportion or parameter with the physical condition being pre-established, and then comparing the value with the correlation criterion, and the skin metabolites collected from the skin surface of the subject are skin metabolites obtained by collecting a skin metabolite on the skin surface by using the aforementioned adhesive tape for collecting skin metabolites according to the present invention.

This enables various physical conditions to be accurately evaluated. Further, certain physical conditions which have been traditionally recognized in a collective manner may be subdivided by virtue of the abundance proportion or parameter of a skin metabolite, thereby improving a physical condition of a subject in a more reliable fashion. In addition, use of the aforementioned adhesive tape for collecting skin metabolites provides satisfactory collection yields of skin metabolites on the skin surface, and thus enables evaluation of physical conditions with high accuracy.

Further, the adhesive tape for collecting skin metabolites according to the present invention can be used for a method of presenting information to a subject. The method of presenting information to a subject is a method including the steps of: evaluating a physical condition of the subject by the aforementioned method of evaluating a physical condition of a subject, and presenting, when the physical condition is evaluated to be outside a target condition, information registered in a data base as an ingredient capable of directing the value of the abundance proportion or the parameter toward a numerical range of the abundance proportion or parameter obtained based on the correlation criterion when the physical condition is within the target condition. This enables information for improving a physical condition and the like to be personalized and presented to a subject. It is noted that the term "information" as used herein encompasses the identity, directions, and dosage of an ingredient or a formulation containing the ingredient, the way and extent of expected improvement in a physical condition, and an adverse effect of the ingredient or formulation.

In the method of presenting information to a subject, the followings are preferably further included: a step of re-evaluating the physical condition of the subject after consumption of the ingredient by the aforementioned method of evaluating a physical condition of a subject; and updating the information based on the extent of improvement in the physical condition after the re-evaluation. Thereby, the information in the data base can be improved, leading to more reliable improvement of a physical condition and others.

There is no particular limitation for the timing of re-evaluation, but, for example, the re-evaluation may be performed when a period of time likely enough to improve a physical condition in response to the use of an ingredient has passed, which may be included in the information in the data base.

Furthermore, disclosed herein, although not forming part of the present invention, is the use of the adhesive tape for collecting skin metabolites in a method of screening for a substance capable of improving or preventing a physical condition. The method of screening for a substance capable of improving or preventing a physical condition includes the step of: selecting a candidate substance based on how the candidate substance changes a value of an abundance proportion of a skin metabolite on a skin surface or a parameter based on the abundance proportion, a correlation criterion of the abundance proportion or parameter with the physical condition being pre-established, and skin metabolites collected from the skin surface are skin metabolites obtained by collecting a skin metabolite on the skin surface of the subject by using the aforementioned adhesive tape for collecting skin metabolites. This can likely increase a probability of screening for a substance capable of improving or preventing a physical condition.

It is noted that the term "screening" means that the probability of the presence of a substance capable of improving or preventing a physical condition is higher in the group of candidate substances after the screening method than in those before the screening method, but a candidate substance may not necessarily be actually capable of improving or preventing a physical condition. Therefore, it is preferred to further perform a step of examining whether a screened candidate substance can actually improve or prevent a physical condition.

The aforementioned methods will be more specifically described.

There is no particular limitation for the parameter as long as it is based on the abundance proportion of a skin metabolite. The parameter may be the difference, sum, product, ratio, exponent, or logarithm of the abundance proportions of a plurality of skin metabolites, or a combination thereof. In particular, the parameter may be the ratio, difference, or a combination thereof. Alternatively, the parameter may be a diversity index of skin metabolites. Such parameters may be each a parameter correlating with a coordinate axis when data for specimens in a population are plotted on a coordinate space by using a technique of dimensionality reduction (for example, principal coordinate analysis, principal component analysis).

There is no particular limitation for the physical condition as long as it shows correlation with the abundance proportion of a skin metabolite on a skin surface or a parameter based on the abundance proportion, but it can be a condition related to any part such as skin, hair, teeth, urine, bone, internal organs, blood, and nerve. Skin conditions include skin age; oily skin; pimpled skin; freckles, texture, yellowishness, dryness, shininess, spots, dullness, sagging, wrinkles, resilience conditions, and pore conditions of the skin; yellowish skin; and the like. Hair conditions include thin hair, hair loss, and the like. Teeth conditions include periodontal disease, alveolar pyorrhea, and the like. Conditions of bone, internal organs, blood, urine, and nerve include liver functions, high uric acid, knee pain, osteoporosis, autonomic imbalance, diabetes, and the like. In addition, the physical condition encompasses menopause which may affect female hormone and aesthetic properties.

The term "correlation criterion" refers to a criterion which can qualitatively or quantitatively determine the goodness or seriousness of a physical condition to be evaluated based on the value of the aforementioned abundance proportion or parameter determined for skin metabolites.

Many of physical conditions may correlate with not only skin metabolites but also an extra phenomenon (for example, actual age). For this reason, an effect from at least one phenomenon known to correlate with a physical condition is preferably removed from the correlation criterion. This can reduce an effect from the presence or absence and the extent of the aforementioned extra phenomenon about a subject, enabling more accurate evaluation of a physical condition based on the value of the abundance proportion or parameter of skin metabolites. Further, according to the method of presenting information to a subject, more suitable information can be presented considering the presence or absence and the extent of the above extra phenomenon about a subject. For this reason, the presence or absence and the extent of the above extra phenomenon about the subject is preferably input in addition to the abundance proportion or parameter. Further, in the method of screening for a substance capable of improving or preventing a physical condition, a substance which may improve or prevent a physical condition tends to be obtained more easily regardless of the above extra phenomenon about a user.

Moreover, the correlation criterion may be created for a sub-population into which a population is categorized according to at least one selected from the group consisting of age, sex, residential area, nationality, and race. By this, a physical condition can be accurately evaluated even considering the age, sex, residential area, nationality, race, and the like of a subject. Further, according to the method of presenting information to a subject, more suitable information can be presented considering the age, sex, residential area, nationality, and race of the subject. For this reason, at least one selected from the group consisting of the age, sex, residential area, nationality, and race of a subject is preferably input in addition to the abundance proportion or parameter. Moreover, in the method of screening for a substance capable of improving or preventing a physical condition, a substance which may improve or prevent a physical condition and be suitable for the age, sex, residential area, nationality, and race of a user tends to be obtained more easily.

There is no particular limitation for the location from which skin metabolites are collected, but it may be the face, scalp, armpit, navel, and the like. Among these, the face, in particular the forehead can be mentioned. The location from which skin metabolites are collected is preferably a skin surface having a sebum level of 75 or higher. This is because the aforementioned adhesive tape for collecting skin metabolites can provide highly improved collection yields of skin metabolites on a skin surface having a sebum level of 75 or higher. Needless to say, the location from which skin metabolites are collected may have a sebum level of lower than 75. The sebum level of a skin surface of a subject varies depending on the environment and the body condition or the like. Thus, collection can be performed for a wide variety of subjects even when the location from which skin metabolites are collected is set to a skin surface having a sebum level of 75 or higher. It is noted that a location from which a sample is collected for a subject preferably coincides with a location from which samples were collected for a population where a correlation criterion was created.

For determination of each value for skin metabolites collected from a skin surface of a subject, it is preferred to calculate it as a value of the amount of the skin metabolite collected from a skin surface of a subject by using the aforementioned adhesive tape for collecting skin metabolites according to the present invention minus the amount of the skin metabolite derived from bacteria concomitantly collected. The amounts of skin metabolites derived from bacteria to be collected are much smaller than those of skin metabolites to be collected, and hence values for skin metabolites collected may be directly used for evaluation of a physical condition. For details, most of soluble proteins collected with the adhesive tape for collecting skin metabolites are inferred to be proteins derived from human skin cells. This is because the amount of total protein derived from bacteria are roughly calculated to be 0.1 µg or less per piece of the adhesive tape for collecting skin metabolites from the amounts of bacteria adhered to the adhesive tape for collecting skin metabolites (determined by qPCR measurement for 16S rRNA). For this calculation, known values were used: the number of the 16S rRNA gene contained in one bacterial cell was assumed to be about 4, and the total amount of proteins contained therein was assumed to be about 0.1 pg.

Specifically, the adhesive layer of the adhesive tape for collecting skin metabolites is contacted with a skin surface, and the adhesive tape is then detached from the skin surface to collect skin metabolites present on the skin surface while the skin metabolites remain adhering to the adhesive layer (the detachment step). Thereby, amongst the entire skin surface metabolites, those present on a skin surface with which the adhesive layer makes contact can be allowed to adhere to and collected at a surface of the adhesive layer of the adhesive tape for collecting skin metabolites while maintaining the original distribution. Advantageously, the aforementioned method can eliminate a risk of variations in the data of skin metabolites due to varied swabbing force and varied swabbing area in the method of swabbing a skin surface with a swab. Further, the skin metabolites of a skin surface can be truly copied. Subsequently, the adhesive layer having the skin metabolites adhered is allowed to make contact with a liquid (for example, PBS: phosphate-buffered saline) and, as necessary, treated with ultrasonication or the like to extract the skin metabolites (the extraction step). Therefore, a solution, from which skin metabolites can be determined, can be prepared very rapidly from the adhesive member having the skin metabolites adhered as compared with a method of sorting substances which have adhered to the surface of an adhesive layer using a magnifying glass and the like to prepare a sample. This can minimize a change in the skin metabolites during the course of sample preparation. Then, the extracted skin metabolites can be analyzed with a commercially available soluble protein measurement kit and the like to simply and uniformly determine the amounts of soluble proteins and the like present on the skin surface. There is also no particular limitation for the means for extracting skin metabolites and the means for analyzing skin metabolites.

### EXAMPLES

Now, the present invention will be described in more detail on the basis of Examples..

### <<Adhesive tape for collecting skin indigenous microorganisms>>

### <Example 1>

### (Collection of microbial flora and measurement of total DNA content)

Collection of a microbial flora was carried out for subjects A to E. Collection of a microbial flora and measurement of total DNA content were carried out according to the flow chart shown in Fig. 2.

### - Contact step S1 and detachment step S2

An adhesive tape 1 for collecting skin indigenous microorganisms, shown in Fig. 1, is prepared. The adhesive tape 1 for collecting skin indigenous microorganisms has a structure in which an adhesive layer 3 including an adhesive is provided on a tape base material 2 as a thin film base. Therefore, it can adhere to the human skin as in a common adhesive bandage and a dressing tape, facilitating the contact step S1. The adhesive tape 1 for collecting skin indigenous microorganisms, which was attached to a release paper (not shown) and enclosed in a re-sealable aluminum bag along with a desiccating agent, was sent to a subject along with latex gloves. A subject was instructed to wear the latex gloves immediately after getting up before face cleansing, and then open the aluminum bag to take out the release paper to which the adhesive tape 1 for collecting skin indigenous microorganisms was attached, and then remove the adhesive tape 1 for collecting skin indigenous microorganisms, and apply it on the forehead. Subsequently, the adhesive tape 1 for collecting skin indigenous microorganisms was allowed to adhere for 5 minutes, and then removed from the forehead, and placed back on the release paper. This was placed in the aluminum bag along with the desiccating agent, and the bag was then re-sealed. Subsequently, it was stored frozen until DNA analysis tests were performed. Table 1 shows the types of the base material and adhesive of the adhesive tape 1 for collecting skin indigenous microorganisms.

### - Extraction step S3

DNA was extracted directly from the adhesive tape 1 for collecting skin indigenous microorganisms in accordance with the method by Morita et al. (Microbes Environ. Vol. 22, No. 3, 214-222, 2007) to prepare a DNA solution. Then, the total DNA content of the DNA solution was measured. Specifically, 1/20 of the volume of the DNA solution was prepared as a specimen sample, with which copy number analysis for the 16S rRNA gene was performed by using a real-time PCR apparatus (Product name: StepOnePlus (TM), Applied Biosystems) with real-time PCR reagents (Product name: KAPA SYBR (R) FAST qPCR Kit, KAPA Biosystems, Inc.) containing KAPA SYBR (R) DNA polymerase enzyme. A calibration curve was prepared by using DNA fragments having an amplification efficiency similar to that of the target molecule as an internal standard.

### (Measurement of sebum level)

For each of the subjects A to E, the sebum level of the forehead was measured before face cleansing on the day of collecting a microbial flora. First, conditioning was performed at a room temperature of 25°C ± 1°C for 20 minutes. After conditioning, the sebum level, the amount of sebum secreted on a skin surface, was measured with a sebmeter (Product name: Sebmeter SM815, Courage + Khazaka electronic GmbH). Specifically, the sebmeter was pressed onto the prominence of the right zygomatic bone for 30 seconds, thereby measuring the sebum level of the contact part three times, and the average of the three measurements was employed as the sebum level. When there was a lentigo or inflammation at a position for measurement, measurement was performed at another position. Sebum levels are classified by skin type, and on the cheek, those of 70 or lower indicate "dry or inadequate sebum", those of 70 to 180 indicate "normal", and those of 180 or higher indicate "oily". The results showed that the serum levels of the subjects A, B, C, D, and E were 146.3, 137.0, 104.7, 82.7, and 76.3, respectively.

### (Measurement of adhesive strength of adhesive tape 1 for collecting skin indigenous microorganisms)

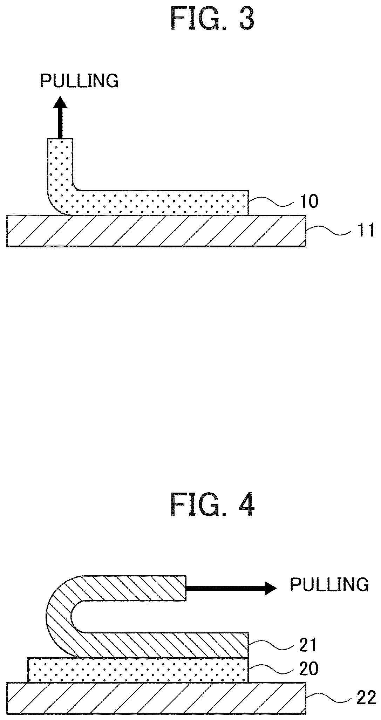

A test piece 10 of 12 mm in width was prepared from the adhesive tape 1 for collecting skin indigenous microorganisms. The adhesive strength of the test piece 10 was measured in accordance with JIS Z 0237: 2009. Specifically, the test piece 10 prepared from the adhesive tape 1 for collecting skin indigenous microorganisms, the adhesive tape including a base material 1 and an adhesive layer 3, was laminated on a Bakelite plate as an adherend 11 by one reciprocal movement of a 2-kg roller with the adhesive layer 3 facing the adherend 11. Subsequently, the adhesive strength was measured, as illustrated in Fig. 3, by using a tensile tester according to JIS B7721: 2009 at a pulling speed of 300 mm/min and a pulling angle of 90°.

### (Measurement of peel strength of adhesive tape 1 for collecting skin indigenous microorganisms)

A test piece 20 of 12 mm in width was prepared from the adhesive tape 1 for collecting skin indigenous microorganisms. The peel strength of the test piece 20 was measured in accordance with JIS Z 0237: 2009. Specifically, the test piece 20 prepared from the adhesive tape 1 for collecting skin indigenous microorganisms, the adhesive tape including a base material 1 and an adhesive layer 3, was applied onto fine paper subjected to silicone treatment as release paper 21 with the adhesive layer 3 facing the silicone-treated surface of the release paper 21. Subsequently, the peel strength was measured, as illustrated in Fig. 4, on an auxiliary plate 22 by using a tensile tester according to JIS B7721: 2009 at a pulling speed of 300 mm/min and a pulling angle of 180°.

### <Comparative Examples 1 to 6>

The same operations as in Example 1 were performed, except that the adhesive tape 1 for collecting skin indigenous microorganisms was modified as shown in Table 1. The adhesive tape for collecting skin indigenous microorganisms in Comparative Example 5 was obtained by applying the adhesive tape for collecting skin indigenous microorganisms in Comparative Example 4 onto both surfaces of the adhesive tape for collecting skin indigenous microorganisms in Example 1.

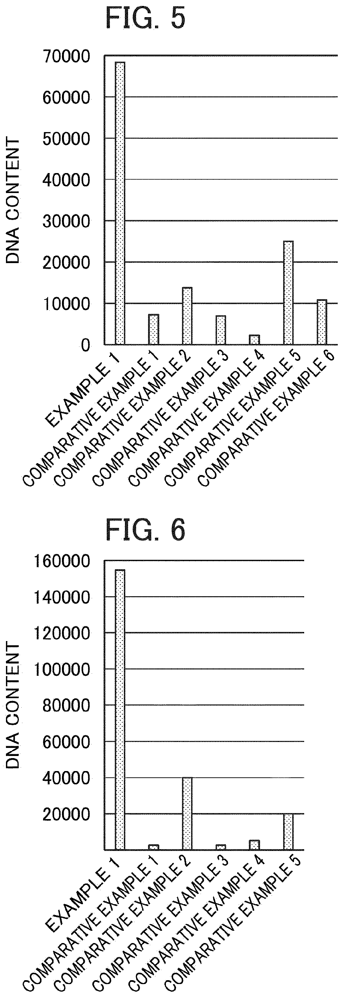

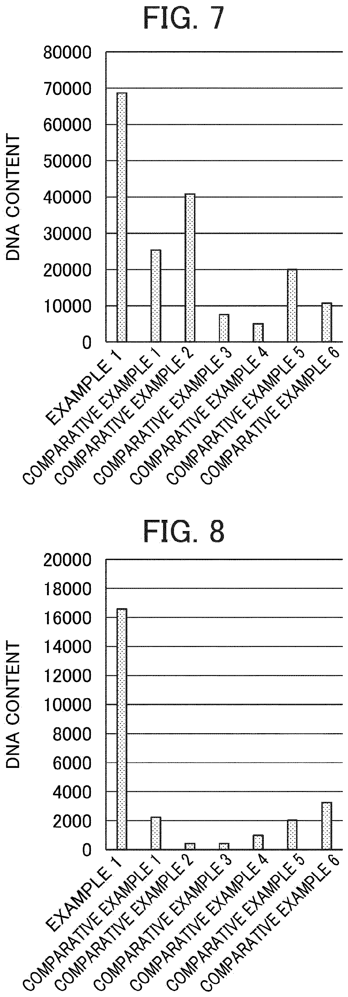

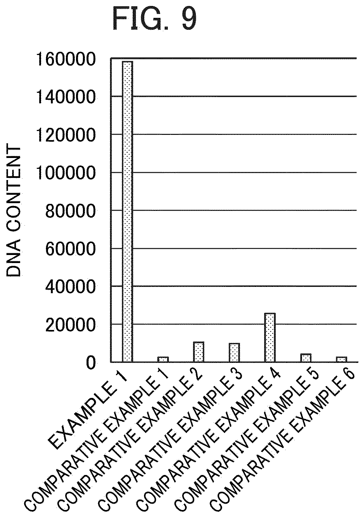

Table 1 shows results of measurement of the adhesive strength and peel strength of the adhesive tape 1 for collecting skin indigenous microorganisms and the thickness of the adhesive tape 1 for collecting skin indigenous microorganisms. For total DNA content, Figs. 5, 6, 7, 8, and 9 show the results of measurement for the subjects A, B, C, D, and E, respectively.

These results showed that, as demonstrated in Table 1 and Figs. 5 to 9, Example 1, using an adhesive tape for collecting skin indigenous microorganisms, the adhesive tape including an adhesive layer containing a rubber adhesive and having an adhesive strength of 10 N/12 mm or more and a peel strength of 0.25 N/mm or more, exhibited significantly higher total DNA contents than Comparative Examples 1 to 6 exhibited.

**[Table 1]**

| | Example 1 | Comparative Example 1 | Comparative Example 2 | Comparative Example 3 | Comparative Example 4 | Comparative Example 5 | Comparative Example 6 |
|---|---|---|---|---|---|---|---|
| Base material | cotton cloth | polyester nonwoven fabric | polyolefin film | vinyl chloride film | polyurethane film | polyurethane film | cellophane |
| Adhesive layer | natural rubber | acrylic | synthetic rubber | acrylic | acrylic | acrylic | natural rubber |
| Thickness (mm) | 0.26 | 0.35 | 0.13 | 0.11 | 0.015 | 0.015 | 0.05 |
| Peel strength (N/12mm) | 0.30 | 0.22 | 0.06 | 0.05 | - | - | 0.30 |
| Adhesive strength (N/12mm) | 13.8 | 4.0 | 8.2 | 4.9 | - | - | 4.7 |

### <<Adhesive tape for collecting skin metabolites>>

### (Collection of soluble proteins and measurement of amount of soluble proteins)

Collection of soluble proteins was carried out for the subjects A and C to E through steps S1 and S2 shown below, and soluble proteins were extracted to quantify the amounts of the soluble proteins through a step S3 shown below.

### - Contact step S1 and detachment step S2

The adhesive tape 1 for collecting skin indigenous microorganisms, which was used in Example 1 above, is employed as an adhesive tape for collecting skin metabolites. The adhesive tape for collecting skin metabolites (the adhesive tape 1 for collecting skin indigenous microorganisms) has a structure in which an adhesive layer including an adhesive is provided on a tape base material as a thin film base, as described above. Therefore, it can adhere to the human skin as in a common adhesive bandage and a dressing tape, facilitating the contact step S1 easily. The adhesive tape for collecting skin metabolites, which was attached to a release paper and enclosed in a re-sealable aluminum bag along with a desiccating agent, was sent to a subject along with latex gloves. A subject was instructed to wear the latex gloves immediately after getting up before face cleansing, and then open the aluminum bag to take out the release paper to which the adhesive tape for collecting skin metabolites was attached, and then remove the adhesive tape for collecting skin metabolites, and apply it on the forehead. Subsequently, the adhesive tape for collecting skin metabolites was allowed to adhere for 5 minutes, and then removed from the forehead, and placed back on the release paper. This was placed in the aluminum bag along with the desiccating agent, and the bag was then re-sealed. Subsequently, it was stored frozen until measurement of the amount of soluble proteins was performed.

### - Extraction step S3

The adhesive tape for collecting skin metabolites after the contact step S1 and detachment step S2 and an adhesive tape for collecting skin metabolites without any attached skin metabolite, as a control, were each put in a container containing 2 mL of PBS (FUJIFILM Wako Pure Chemical Corporation), and the containers were subjected to ultrasonic disintegration treatment for 15 minutes. The ultrasonic disintegration treatment was performed by using an ultrasonic disintegrator (Bioruptor (R) Pico, Diagenode, S. A.) in 30 cycles each including power-on for 15 seconds and power-off for 15 seconds.

### - Calculation of amounts of soluble proteins derived from human skin cells

The total amount of soluble proteins in the solution obtained by ultrasonic disintegration treatment in the extraction step S3 was measured by using the measurement kit Micro BCA Protein Assay Kit (Thermo Fisher Scientific) and the measurement apparatus Nanodrop ND-1000 (Thermo Fisher Scientific) under a condition of 562 nm spectrophotometer. In addition, the amount of soluble proteins derived from bacteria concomitantly collected with the adhesive tape for skin metabolites was calculated as Copy number of 16S rRNA gene / Number of 16S rRNA possessed by unit bacterial cell (3.5) × Amount of proteins possessed by unit bacterial cell (0.1 pg). The results showed that the amount of soluble proteins derived from bacteria per piece of a tape was as little as 0.04 µg or less for any of Example and Comparative Examples. From the total amount of soluble proteins extracted from the adhesive tape for collecting skin metabolites after the contact step S1 and the detachment step S2, the amount of proteins extracted from the adhesive tape for collecting skin metabolites without any attached skin metabolite and the amount of soluble proteins estimated to be derived from bacteria were subtracted, and the resulting value was employed as the amount of soluble proteins derived from human skin cells.

### (Measurement of sebum level)

For each of the subjects A and C to E, the sebum level of the forehead was measured before face cleansing on the day of collecting skin metabolites. First, conditioning was performed at a room temperature of 25°C ± 1°C for 20 minutes. After conditioning, the sebum level, the amount of sebum secreted on a skin surface, was measured with a sebmeter (Product name: Sebmeter SM815, Courage + Khazaka electronic GmbH). Specifically, the sebmeter was pressed onto the prominence of the right zygomatic bone for 30 seconds, thereby measuring the sebum level of the contact part three times, and the average of the three measurements was employed as the sebum level. When there was a lentigo or inflammation at a position for measurement, measurement was performed at another position. Sebum levels are classified by skin type, and on the cheek, those of 70 or lower indicate "dry or inadequate sebum", those of 70 to 180 indicate "normal", and those of 180 or higher indicate "oily". The results showed that the serum levels of the subjects A, C, D, and E were 146.3, 104.7, 61.3, and 74.0, respectively.

The same operations were performed, except that the adhesive tape for collecting skin metabolites was changed from the adhesive tape for collecting skin indigenous microorganisms, which was used in Example 1, to each of those in Comparative Examples 1, 2, and 6 shown in Table 1.

For the total amount of soluble proteins extracted and amount of soluble proteins estimated to be derived from human skin cells, Tables 2, 3, 4, and 5 show the results for the subjects A, C, D, and E, respectively.

**[Table 2]**

| | Total amount of soluble proteins extracted per piece of tape (µg) | Amount of soluble proteins derived from human skin cells per piece of tape (µg) |
|---|---|---|
| Example 1 | 214 | 210 |
| Comparative Example 1 | 20 | 16 |
| Comparative Example 2 | 160 | 124 |
| Comparative Example 6 | 108 | 67 |

**[Table 3]**

| | Total amount of soluble proteins extracted per piece of tape (µg) | Amount of soluble proteins derived from human skin cells per piece of tape (µg) |
|---|---|---|
| Example 1 | 124 | 120 |
| Comparative Example 1 | 26 | 22 |
| Comparative Example 2 | 110 | 74 |
| Comparative Example 6 | 86 | 45 |

**[Table 4]**

| | Total amount of soluble proteins extracted per piece of tape (µg) | Amount of soluble proteins derived from human skin cells per piece of tape (µg) |
|---|---|---|
| Example 1 | 138 | 134 |
| Comparative Example 1 | 28 | 24 |
| Comparative Example 2 | 108 | 72 |
| Comparative Example 6 | 88 | 47 |

**[Table 5]**

| | Total amount of soluble proteins extracted per piece of tape (µg) | Amount of soluble proteins derived from human skin cells per piece of tape (µg) |
|---|---|---|
| Example 1 | 96 | 92 |
| Comparative Example 1 | 40 | 36 |
| Comparative Example 2 | 114 | 78 |
| Comparative Example 6 | 52 | 11 |

These results showed that, as demonstrated in Table 1 and Figs. 5 to 9, Example 1, using an adhesive tape for collecting skin indigenous microorganisms, the adhesive tape including an adhesive layer containing a rubber adhesive and having an adhesive strength of 10 N/12 mm or more and a peel strength of 0.25 N/mm or more, exhibited significantly higher total DNA contents than Comparative Examples 1 to 6 exhibited. In addition, as demonstrated in Tables 2 to 5, Example 1, using an adhesive tape for collecting skin metabolites, the adhesive tape including an adhesive layer containing a rubber adhesive and having an adhesive strength of 10 N/12 mm or more and a peel strength of 0.25 N/mm or more, exhibited significantly larger amounts of soluble proteins than Comparative Examples 1, 2, and 6 exhibited.

### EXPLANATION OF REFERENCE NUMERALS

1 Adhesive tape for collecting skin indigenous microorganisms
2 Base material
3 Adhesive layer

## Claims

1. An adhesive tape (1) for collecting skin indigenous microorganisms or skin metabolites on a skin surface, the adhesive tape comprising:
a base material (2); and
an adhesive layer (3) provided on at least a portion of a surface of the base material (2), wherein
the adhesive layer (3) contains an adhesive, and the adhesive contains a rubber adhesive,
the adhesive tape (1) has an adhesive strength of 10.0 N or more when a 12 mm-wide test piece thereof is pressure-bonded to a Bakelite plate by one reciprocation of a 2 kg roller and then measured for adhesive strength at a pulling speed of 300 mm/min and a pulling angle of 90° according to JIS Z 0237: 2009, and, wherein
the adhesive tape (1) has a peel strength of 0.25 N or more when a 12 mm-wide test piece thereof is measured using a silicone-treated fine paper sheet as a release sheet and measured for peel strength at a pulling speed of 300 mm/min and a pulling angle of 180° according to JIS Z 0237: 2009.

2. The adhesive tape (1) for collecting skin indigenous microorganisms or skin metabolites according to claim 1, further comprising skin indigenous microorganisms and/or skin metabolites.

3. The adhesive tape (1) for collecting skin metabolites according to claim 1, wherein the skin metabolite is a soluble protein.

4. A method of analysing a sample from a subject, the method comprising the step of:
determining a value of an abundance proportion of a skin indigenous microorganism or a skin metabolite on a skin surface of the subject among a skin microbial flora or skin metabolites, or a parameter based on the abundance proportion, a correlation criterion of the abundance proportion or parameter with a physical condition being preestablished, and then comparing the value with the correlation criterion,
in a sample collected from the skin surface using the adhesive tape (1) for collecting skin indigenous microorganisms or skin metabolites according to any one of claims 1 to 3.

5. A method of presenting information to a subject, the method comprising the steps of:
analysing a sample from a subject by the method according to claim 4, and
presenting, when the physical condition is evaluated to be outside a target condition, information registered in a data base as an ingredient capable of directing the value of the abundance proportion or the parameter toward a numerical range of the abundance proportion or parameter obtained based on the correlation criterion when the physical condition is within the target condition.

6. The method according to claim 5, further comprising the steps of: re-analysing the sample from a subject by the method according to claim 4 after consumption of the ingredient, and
updating the information based on the extent of improvement in the physical condition after the re-analysis.

## Patentansprüche

1. Klebeband (1) zum Sammeln von indigenen Hautmikroorganismen oder Hautmetaboliten auf einer Hautfläche, wobei das Klebeband Folgendes umfasst:
ein Trägermaterial (2) und
eine Klebschicht (3), die zumindest auf einem Abschnitt einer Fläche des Trägermaterials (2) angeordnet ist, wobei
die Klebschicht (3) einen Klebstoff enthält und der Klebstoff einen Kautschukklebstoff enthält;
das Klebeband (1) eine Klebkraft von 10,0 N oder mehr aufweist, wenn ein 12 mm breites Teststück davon mittels eines Hin- und Herbewegens einer 2-kg-Walze an eine Bakelitplatte haftgeklebt und dann seine Klebkraft bei einer Abziehgeschwindigkeit von 300 mm/min und einem Abziehwinkel von 90° gemäß JIS Z 0237: 2009 gemessen wird und wobei
das Klebeband (1) eine Abschälkraft von 0,25 N oder mehr aufweist, wenn ein 12 mm breites Teststück davon mittels eines silikonbehandelten Feinpapierbogens als Trennbogen gemessen wird und seine Abschälkraft bei einer Abziehgeschwindigkeit von 300 mm/min und einem Abziehwinkel von 180° gemäß JIS Z 0237: 2009.

2. Klebeband (1) zum Sammeln von indigenen Hautmikroorganismen oder Hautmetaboliten nach Anspruch 1, das weiterhin indigene Hautmikroorganismen und/oder Hautmetaboliten umfasst.

3. Klebeband (1) zum Sammeln von Hautmetaboliten nach Anspruch 1, wobei der Hautmetabolit ein lösliches Protein ist.

4. Verfahren zum Analysieren einer Probe von einem Individuum, wobei das Verfahren den folgenden Schritt umfasst:
das Bestimmen eines Wertes eines Abundanzanteils eines indigenen Hautmikroorganismus oder eines Hautmetaboliten auf einer Hautfläche des Individuums von einer Hautmikrobenflora oder Hautmetaboliten oder eines auf dem Abundanzanteil basierenden Parameters, wobei ein Korrelationskriterium des Abundanzanteils oder Parameters mit einem physischen Zustand zuvor festgelegt wird, und dann das Vergleichen des Wertes mit dem Korrelationskriterium in einer Probe, die unter Verwendung des Klebebands (1) zum Sammeln von indigenen Hautmikroorganismen oder Hautmetaboliten nach einem der Ansprüche 1 bis 3 gesammelt wird.

5. Verfahren, um einem Individuum Informationen zu präsentieren, wobei das Verfahren die folgenden Schritte umfasst:
das Analysieren einer Probe von einem Individuum mittels des Verfahrens nach Anspruch 4 und,
wenn bestimmt wird, dass der physische Zustand außerhalb einer Zielbedingung liegt, das Präsentieren von in einer Datenbank registrierten Informationen als einen Inhaltsstoff, der dazu fähig ist, den Wert des Abundanzanteils oder des Parameters zu einem Zahlenbereich des Abundanzanteils oder des basierend auf dem Korrelationskriterium erhaltenen Parameters zu leiten, in dem der physische Zustand innerhalb der Zielbedingung liegt.

6. Verfahren nach Anspruch 5, das weiterhin die folgenden Schritte umfasst: das erneute Analysieren der Probe von einem Individuum mit dem Verfahren nach Anspruch 4 nach dem Verbrauch des Inhaltsstoffs und
das Aktualisieren der Informationen basierend auf dem Ausmaß der Verbesserung des physischen Zustands nach der erneuten Analyse.

## Revendications

1. Ruban adhésif (1) pour prélèvement des micro-organismes indigènes de la peau ou des métabolites de la peau sur une surface de la peau, le ruban adhésif comprenant :
un matériau de base (2) ; et
une couche d'adhésif (3) fournie sur au moins une partie d'une surface du matériau de base (2), dans lequel
la couche d'adhésif (3) contient un adhésif, et l'adhésif contient un adhésif de caoutchouc,
le ruban adhésif (1) présente une force d'adhérence de 10,0 N ou plus lorsqu'une éprouvette de celui-ci de 12 mm de large est collée par pression sur une plaque de bakélite par un mouvement de va-et-vient d'un rouleau de 2 kg et ensuite mesurée pour la force d'adhérence à une vitesse de traction de 300 mm/min et un angle de traction de 90° selon la norme JIS Z 0237: 2009, et, dans lequel
le ruban adhésif (1) présente une résistance au pelage de 0,25 N ou plus lorsqu'une éprouvette de celui-ci de 12 mm de large est mesurée à l'aide d'une feuille de papier fin traitée par une silicone comme feuille de libération et mesurée pour la résistance au pelage à une vitesse de traction de 300 mm/min et un angle de traction de 180° selon la norme JIS Z 0237: 2009.

2. Ruban adhésif (1) pour prélèvement des micro-organismes indigènes de la peau ou des métabolites de la peau selon la revendication 1, comprenant en outre des micro-organismes indigènes de la peau et/ou des métabolites de la peau.

3. Ruban adhésif (1) pour prélèvement des métabolites de la peau selon la revendication 1, dans lequel le métabolite de la peau est une protéine soluble.

4. Procédé d'analyse d'un échantillon provenant d'un sujet, le procédé comprenant l'étape de :
détermination d'une valeur d'une proportion d'abondance d'un micro-organisme indigène de la peau ou d'un métabolite de la peau sur une surface de la peau du sujet parmi une flore microbienne de la peau ou des métabolites de la peau ou un paramètre basé sur la proportion d'abondance, un critère de corrélation de la proportion ou du paramètre d'abondance avec une condition physique étant préétabli, et ensuite comparaison de la valeur avec le critère de corrélation dans un échantillon prélevé de la surface de la peau en utilisant le ruban adhésif (1) pour prélèvement des micro-organismes indigènes de la peau ou des métabolites de la peau selon l'une quelconque des revendications 1 à 3.

5. Procédé de présentation d'informations à un sujet, le procédé comprenant les étapes de :
analyse d'un échantillon provenant d'un sujet par le procédé selon la revendication 4, et
présentation, lorsque la condition physique est évaluée pour être en dehors d'une condition cible, d'informations enregistrées dans une base de données en tant qu'ingrédient capable de diriger la valeur de la proportion ou du paramètre d'abondance vers une plage numérique de la proportion ou du paramètre d'abondance obtenue sur la base du critère de corrélation lorsque la condition physique est dans la condition cible.

6. Procédé selon la revendication 5, comprenant en outre les étapes de : réanalyse de l'échantillon provenant d'un sujet par le procédé selon la revendication 4 après consommation de l'ingrédient, et mise à jour des informations en fonction de l'étendue de l'amélioration de la condition physique après la réanalyse.
